# FASCICULE DE BREVET EUROPEEN

(11) **EP 1 749 515 B1**
(45) Date de publication et mention de la délivrance du brevet: **19.08.2009**
(21) Numéro de dépôt: 06116464.6
(22) Date de dépôt: 30.06.2006
(51) Int. Cl.: A61K 8/86, A61K 8/90, A61K 8/73, A61Q 3/02

(54) **Vernis à ongles comprenant un polymère bloc polyoxyethyléne/polyoxypropylène**
Nagellack enthaltend ein Polyoxyethylen/polyoxypropylen-blockpolymer
Nail coating comprising a polyoxyethylene/polyoxypropylene block polymer

(30) Priorité: 29.07.2005 FR 0508179
(43) Date de publication de la demande: 07.02.2007
(73) Titulaire: L'ORÉAL, 75008 Paris (FR)
(72) Inventeur: Ilekti, Philippe, 94700 Maison-Alfort (FR)
(74) Mandataire: Duvert, Sandra

(56) Documents cités:
- EP-A- 1 125 573
- EP-A- 1 595 524
- US-A- 4 158 053
- US-A- 4 229 227
- US-A- 5 547 660

## Description

La présente invention a pour objet un vernis à ongles comprenant un copolymère bloc polyoxyalkylène selon la revendication 1. L'invention a également pour objet un procédé de maquillage ou de soin des ongles.

La composition de vernis à ongles peut être employée comme base pour vernis ou "base-coat" en terminologie anglo-saxonne, comme produit de maquillage des ongles, comme composition de finition, encore appelée "top-coat", à appliquer sur le produit de maquillage des ongles ou bien encore comme produit de soin cosmétique des ongles. Ces compositions peuvent s'appliquer sur les ongles d'êtres humains ou bien encore sur des faux ongles.

Les brevets US 5,547,660, US 4,158,053, et US 4,229,227, et les demandes EP 1 125 573 et EP 1 595 524 décrivent des compositions de vernis à ongles. Les compositions de vernis à ongles comprennent en général des particules solides telles que les pigments, les nacres, les charges, qui sont en dispersion dans le milieu continu aqueux ou le milieu solvant organique de la composition.
Or ces particules ont tendance à sédimenter au cours du temps, du fait de leur densité qui est supérieure à celle du milieu continu dans lequel elles sont dispersées. Cette sédimentation se traduit par une modification de l'aspect macroscopique de la composition, et en particulier, dans le cas de vernis à ongles colorés, par une hétérogénéité de la couleur du vernis et une détérioration de la tenue du film de vernis sur les ongles.

Afin d'améliorer la stabilisation des compositions, le formulateur dispose d'agents épaississants ou gélifiants tels que par exemple la bentone ; toutefois, l'incorporation de ces agents en une quantité nécessaire à la stabilisation du vernis a tendance à matifier le film de vernis ce qui n'est pas souhaitable dans le cas des vernis à ongles où l'on cherche à obtenir un film brillant sur l'ongle.

Le demandeur a découvert de façon surprenante que l'incorporation dans un vernis à ongles à milieu solvant organique, d'un copolymère bloc polyoxyalkylène particulier permet l'obtention d'une composition de vernis à ongles qui présente une bonne stabilité et une bonne homogénéité de la couleur dans le temps, ainsi que la formation d'un film sur les ongles brillant et présentant une bonne tenue dans le temps.

De façon plus précise, l'invention a pour objet une composition de vernis à ongles comprenant, dans un milieu cosmétiquement acceptable comprenant un milieu solvant organique :
- au moins un copolymère bloc de formule (I) suivante :

   HO-(CH2-CH2-O)n-(CxH2xO)m-(CH2-CH2-O)p-H,

   dans laquelle n,m,p sont indépendamment des nombres entiers allant de 1 à 1000 et x est égal à 3, ledit copolymère bloc étant présent en une teneur d'au moins 0,1% en poids par rapport au poids total de la composition et
- au moins un agent épaississant,
ledit polymère bloc étant un polycondensat tribloc polyéthylèneglycol-polypropylèneglycol-polyéthylèneglycol.

Ces copolymères bloc polyoxyalkylène particuliers permettent de stabiliser la composition selon l'invention et peuvent être formulés en complément d'un gélifiant classique comme la bentone ou la silice, sans avoir l'inconvénient de l'adjonction de grandes quantités de bentone qui entraîne une perte de brillance du film de vernis.

Par "milieu cosmétiquement acceptable", on entend au sens de l'invention un milieu non toxique et susceptible d'être appliqué sur la peau, les phanères ou les lèvres du visage d'êtres humains.

L'invention a aussi pour objet un procédé cosmétique de maquillage ou de soin non thérapeutique des ongles comprenant l'application sur les ongles d'au moins une couche d'une composition de vernis à ongles telle que définie ci-dessus.

### Copolymère bloc

Par copolymère bloc, on entend un polymère comprenant au moins 2 blocs ou séquences distinctes, de préférence au moins 3 séquences distinctes.

Le copolymère bloc de la composition selon l'invention comprend des séquences polyoxyalkylénées et répond en particulier à la formule (1) suivante :

HO―(CH2-CH2-O)n-(CxH2xO)m-(CH2-CH2-O)p-H

avec x = 3 et
dans laquelle n,m,p sont indépendamment des nombres entiers allant de 1 à 1000 ,ledit polymère de l'invention étant un polycondensat tribloc polyéthylèneglycol-polypropylèneglycol-polyéthylèneglycol.

Le copolymère bloc de la composition présente avantageusement un poids moléculaire supérieur ou égal à 1000 g/mol, de préférence supérieur ou égal à 1500 g/mol, mieux supérieur à 2000 g/mol.

A titre de copolymère utilisables dans la composition selon l'invention, on peut citer les polycondensats tribloc polyéthylène glycol / polypropylène glycol / polyéthylène glycol vendus sous les dénominations "SYNPERONIC" comme les "SYNPERONIC PE/ L44", le SYNPERONIC PE/ L64 (13 OE/13OP/13OE) de PM 2900 et "SYNPERONIC PE/F127 "par la société ICI, et leurs mélanges.

Le ou les copolymères blocs sont présents en une teneur supérieure ou égale 0,1% en poids par rapport au poids total de la composition, de préférence supérieure ou égale à 0,5% en poids et mieux supérieure ou égale à 1% en poids.
Ils peu(ven)t par exemple être présent(s) en une quantité allant de 0,1 à 15% en poids par rapport au poids total de la composition, de préférence de 0,5 à 10%, et mieux de 1 à 8% en poids.

### Milieu solvant organique

Le milieu cosmétiquement acceptable de la composition cosmétique selon l'invention comprend un milieu solvant organique comprenant un solvant organique ou un mélange de solvants organiques.

Le solvant organique peut être choisi parmi :
- les cétones liquides à température ambiante tels que méthyléthylcétone, méthylisobutylcétone, düsobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle ;
- les carbonates tel que le carbonate de propylène, le carbonate de diméthyle ,
- les acétals tels que méthylal ;
- et leurs mélanges.

De préférence, le solvant est choisi parmi les esters à chaîne courte ayant de 3 à 8 atomes de carbone au total, tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle et leurs mélanges.

Le milieu solvant organique peut représenter de 10 à 95% en poids, par rapport au poids total de la composition, de préférence de 15% à 80% en poids, et mieux de 20 à 60% en poids.

La composition selon l'invention peut éventuellement comprendre un milieu aqueux, dans ce cas, le milieu aqueux est présent en une teneur inférieure ou égale à 2% en poids par rapport au poids total de la composition, de préférence inférieure ou égale à 1% en poids.

### Polymère filmogène

La composition comprendre avantageusement un polymère filmogène.
Selon la présente invention, on entend par "polymère filmogène", un polymère apte à former à lui seul ou en présence d'un agent auxiliaire de filmification, un film continu et adhérent sur un support, notamment sur les matières kératiniques.
Parmi les polymères filmogènes utilisables dans la composition de la présente invention, on peut citer les polymères synthétiques, de type radicalaire ou de type polycondensat, les polymères d'origine naturelle et leurs mélanges.

Le polymère filmogène peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy ou encore les résines éthyl tosylamide.

Comme polymère filmogène, on peut notamment utiliser la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES ; les résine toluène sulfonamide formaldéhyde "Ketjentflex MS80" de la société AKZO ou "Santolite MHP", "Santolite MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.
Avantageusement, les première et deuxième séquences et du polymère séquencé sont incompatibles l'une avec l'autre.
De tels polymères sont décrits par exemple dans les documents EP 1411069 ou WO04/028488.

Le polymère filmogène additionnel peut être présent dans la composition selon l'invention en une teneur allant de 0,1 % à 60 % en poids, par rapport au poids total de la composition, de préférence allant de 2 % à 40 % en poids, et mieux de 5 % à 25 % en poids.

### Agent auxiliaire de filmification

Pour améliorer les propriétés filmogènes de la composition de vernis à ongles un agent auxiliaire de filmification peut être prévu.
Un tel agent auxiliaire de filmification peut être choisi parmi tous les composés connus de l'homme du métier, comme étant susceptibles de remplir la fonction recherchée, et être notamment choisi parmi les agents plastifiants et les agents de coalescence du ou des polymère(s) filmogène(s).

Ainsi, la composition peut comprendre, en outre, au moins un agent plastifiant et/ou un agent de coalescence. En particulier, on peut citer, seuls ou en mélange, les plastifiants et agents de coalescence usuels, tels que :
- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les polyéthylène glycols, les polypropylène glycols, les copolymères polyéthylène glycols-polypropylène glycols et leurs mélanges, notamment les polypropylène glycols de haut poids moléculaire, ayant par exemple une masse moléculaire allant de 500 à 15000, tels que par exemple
- les esters de glycol ;
- les dérivés de propylène glycol et en particulier le propylène glycol phényléther, le propylène glycol diacétate, le dipropylène glycol éthyléther, le tripropylène glycol méthyléther et le diéthylène glycol méthyléther, le propylène glycol butyléther ;
- des esters d'acides, notamment carboxyliques, tels que des citrates, des phtalates, des adipates, des carbonates, de tartrates, des phosphates, des sébaçates ;
- les esters issus de la réaction d'un acide monocarboxylique de formule R₁₁COOH avec un diol de formule HOR₁₂OH avec R₁₁ et R₁₂, identiques ou différents, représentent une chaîne hydrocarbonée, comprenant de préférence de 3 à 15 atomes de carbone, linéaire, ramifiée ou cyclique, saturée ou insaturée comprenant éventuellement un ou plusieurs hétéroatomes tels que N, O, S, en particulier le monoester résultant de la réaction de l'acide isobutyrique et d'octanediol tel que le triméthyl-2,2,4 pentane diol 1,3, tel que celui commercialisé sous la référence TEXANOL Ester Alcohol par la société Eastman Chemical,
- des dérivés oxyéthylénés, tels que les huiles oxyéthylénées, notamment les huiles végétales, telles que l'huile de ricin ;
- les dimethicone copolyols, notamment à groupements 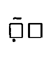 propyl polyoxypropylène et
- leurs mélanges.

### Agent épaississant

La composition peut comprendre, outre le copolymère bloc, un agent épaississant qui peut en particulier être choisi parmi : les silices notamment hydrophobes, telles que celles décrites dans le document EP-A-898960, et par exemple commercialisées sous les références "AEROSIL R812®" par la société Degussa, "CAB-O-SIL TS-530®", "CAB-O-SIL TS-610®", "CAB-O-SIL TS-720®" par la société Cabot, "AEROSIL R972®", "AEROSIL R974®" par la société Degussa ; les argiles telles que la montmorillonite, les argiles modifiées telles que les bentones par exemple, l'hectorite stéaralkonium, la bentonite stéaralkonium , les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.

La proportion totale en agent(s) épaississant(s) dans les compositions selon l'invention peut aller de à 0,01 à 15 % en poids, par rapport au poids total de la composition, de préférence de 0,1 à 10% et mieux de 0,1 à 15% en poids.

Selon un mode de réalisation particulier, la teneur totale en agents épaississants est inférieure ou égale à 5 % en poids, par rapport au poids total de la composition, de préférence inférieure ou égale à 3% en poids.

Selon un mode de réalisation particulier, la composition comprend un agent épaississant choisi parmi les bentones, en une teneur inférieure ou égale à 2% en poids par rapport au poids total de la composition, de préférence inférieure ou égale à 1% en poids.

### Matière colorante

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.
Parmi les pigments organiques, on peut citer le noir de carbone, les pigments de type D & C, et les laques à base de carmin de cochenille, de baryum, strontium, calcium, aluminium.
Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.
Les colorants hydrosolubles sont par exemple le jus de betterave, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.
Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon@) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

### Autres Additifs

La composition peut comprendre, en outre, d'autres ingrédients utilisés couramment dans les compositions cosmétiques. De tels ingrédients peuvent être choisis parmi les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Selon un autre aspect, l'invention a pour objet un produit de vernis à ongles comprenant : i) un récipient délimitant au moins un compartiment, ledit récipient étant fermé par un élément de fermeture et ii) une composition selon l'invention qui est disposée à l'intérieur dudit compartiment.

Le récipient peut être sous toute forme adéquate. Il peut être notamment sous forme d'une bouteille et peut être, au moins pour partie, en un matériau tel que le verre. Toutefois, des matériaux autres que le verre peuvent être utilisés comme les matériaux thermoplastiques tels que le PP ou le PE ou comme un métal.

L'élément de fermeture peut être couplé au compartiment par vissage en position fermée du récipient. Alternativement, le couplage entre l'élément de fermeture et le récipient peut se faire autrement que par vissage, notamment par encliquetage.

Le récipient est de préférence équipé d'un applicateur pouvant être sous forme d'un pinceau constitué d'au moins une touffe de poils. Alternativement, l'applicateur se présente sous forme autre qu'un pinceau, par exemple sous forme d'une spatule ou d'un embout en mousse.

Les exemples qui suivent illustrent l'invention. Sauf indication contraire, les quantités indiquées sont des pourcentages massiques.

### Exemple 1 : Vernis à ongles

| | |
|---|---|
| Nitrocellulose à 30 % d'alcool isopropylique (viscosité: E22 - 1/2 S) | 14 |
| Nitrocellulose à 30 % d'alcool isopropylique (Azur E80 de Bergerac) | 2,45 |
| Résine alkyde glycérophtalique ésterifiée par acides gras ramifiés⁽¹⁾ dans l'acétate d'éthyle à 70 % | 14 |
| Acétyle citrate de tributyle | 2,45 |
| N-éthyl O,P-toluènesulfonamide | 3,42 |
| Hectorite modifiée stéaryl benzyl diméthyl ammonium | 0,7 |
| Oxyde de titane enrobé d'oxyde de polyéthylène (97/3) | 0,047 |
| Laque Red 34 | 0,26 |
| Condensat Oxyde d'éthylène/oxyde de propylène/ Oxyde d'éthylène (130E/300P/13OE)⁽²⁾ | 2,45 |
| Alcool isopropylique | 3,23 |
| Acide citrique, 1 H2O | 0,03 |
| Acétate d'éthyle | 20 |
| Acétate de butyle | Qsp 100 |

| | |
|---|---|
| ⁽¹⁾ commercialisé par SHELL sous le nom de marque CARDURA 30®. ⁽²⁾ commercialisé par UNIQEMA sous la référence SYNPERONIC PE/ L64 | |

On a mesuré la brillance du vernis selon le protocole suivant :
Sur une carte de contraste de marque LENETA et de référence FORM 1A PENOPAC, on étale une couche de 300 µm d'épaisseur humide de la composition dont on cherche à évaluer la brillance moyenne, à l'aide d'un étaleur automatique. La couche recouvre le fond blanc et la fond noir de la carte. On laisse sécher pendant 24 heures sur un banc thermostaté à 30°C puis on procède à la mesure de la brillance à 20° et à 60° sur le fond blanc (3 mesures) et sur le fond noir (3 mesures) à l'aide d'un brillancemètre de marque BYK GARDNER et de référence microTRI-GLOSS.
Le vernis à ongles de l'exemple 1 présente une brillance mesurée à 20° égale à 74 et une brillance à 60° égale à 89.

## Revendications

1. Composition de vernis à ongles comprenant, dans un milieu cosmétiquement acceptable comprenant un milieu solvant organique :
- au moins un copolymère bloc de formule (I) suivante :
HO-(CH2-CH2-O)n-(CxH2xO)m-(CH2-CH2-O)p-H,
dans laquelle n,m,p sont indépendamment des nombres entiers allant de 1 à 1000 et x égal à 3;
ledit copolymère bloc étant présent en une teneur d'au moins 0, 1 % en poids par rapport au poids total de la composition,
ledit copolymère bloc étant un polycondensat tribloc polyéthylène glycol polypropylène glycol / polyéthylène glycol, et
- au moins un agent épaississant.

2. Composition selon la revendication 1, **caractérisée en ce que** le copolymère bloc est présent en une quantité allant de 0,1 à 15% en poids par rapport au poids total de la composition, de préférence de 0,5 à 10%, et mieux de 1 à 8% en poids.

3. Composition selon l'une des revendications précédentes, **caractérisée en ce que** le milieu solvant organique comprend au moins un solvant organique choisi parmi :
- les cétones liquides à température ambiante tels que les méthyléthylcétone, méthylisobutylcétone, diisobutytcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle ;
- les carbonates tel que le carbonate de propylène, le carbonate de diméthyle,
- les acétales tels que le méthylal ;
- leurs mélanges.

4. Composition selon l'une des revendications précédentes, **caractérisée en ce que** le milieu solvant organique représente de 10 à 95% en poids, par rapport au poids total de la composition, de préférence de 15% à 80% en poids, et mieux de 20 à 60% en poids.

5. Composition selon l'une des revendications précédentes, **caractérisée en ce que** l'agent épaississant est choisi parmi les silices, notamment hydrophobes, les argiles telles que la montmorillonite, les argiles modifiées telles que les bentones, les alkyléther de polysaccharides et leurs mélanges.

6. Composition selon l'une des revendications précédentes, **caractérisée en ce que** l'agent épaississant est présent en une teneur allant de 0,01 à 15 % en poids, par rapport au poids total de la composition, de préférence de 0,1 à 10% et mieux de 0,1 à 15% en poids.

7. Composition selon l'une des revendications 1 à 5, **caractérisée en ce que** la teneur totale en agents épaississants est inférieure ou égale à 5 % en poids, par rapport au poids total de la composition, de préférence inférieure ou égale à 3% en poids.

8. Composition selon l'une des revendications précédentes, **caractérisée en ce qu'**elle comprend un agent épaississant choisi parmi les bentones, en une teneur inférieure ou égale à 2% en poids par rapport au poids total de la composition.

9. Composition selon l'une des revendications précédentes, **caractérisée en ce qu'**elle comprend un polymère filmogène.

10. Composition selon la revendication précédente, **caractérisée en en ce que** le polymère filmogène est présente en une teneur allant de 0,1 % à 60 % en poids, par rapport au poids total de la composition, de préférence allant de 2 % à 40 % en poids, et mieux de 5 % à 25 % en poids.

11. Composition selon l'une des revendications précédentes, **caractérisée en ce qu'**elle comprend une matière colorante.

12. Composition selon la revendication précédente**, caractérisée en ce que** la matière colorante est présent en une teneur allant de 0,01 % à 50 % en poids, par rapport au poids de la composition, de préférence de 0,01 % à 30 % en poids.

13. Procédé cosmétique de maquillage ou de soin non thérapeutique des ongles comprenant l'application sur les ongles d'au moins une couche d'une composition de vernis à ongles selon l'une des revendications 1 à 12.

14. Ensemble cosmétique comprenant : i) un récipient délimitant au moins un compartiment, ledit récipient étant fermé par un élément de fermeture et ii) une composition disposée à l'intérieur dudit compartiment, la composition étant conforme à l'une quelconque des revendications 1 à 12.

15. Ensemble cosmétique selon la revendication 14 **caractérisé en ce que** le récipient est formé, au moins pour partie, en verre.

16. Ensemble cosmétique selon la revendication 14 **caractérisé en ce que** le récipient est formé, au moins pour partie, en au moins un matériau autre que le verre, par exemple un matériau thermoplastique ou un métal.

17. Ensemble selon l'une quelconque des revendications 14 à 16 **caractérisé en ce que**, en position fermée du récipient, l'élément de fermeture est vissé sur le récipient.

18. Ensemble selon l'une quelconque des revendications 14 à 16 **caractérisé en ce que,** en position fermée du récipient, l'élément de fermeture est couplé au récipient autrement que par vissage, notamment par encliquetage.

19. Ensemble selon l'une quelconque des revendications 14 à 18 **caractérisé en ce qu'**il comporte un applicateur sous forme d'un pinceau comprenant au moins une touffe de poils.

20. Ensemble selon l'une quelconque des revendications 14 à 18 **caractérisé en ce qu'**il comporte un applicateur différent d'un pinceau, tel qu'une spatule ou un embout en mousse.

## Claims

1. Nail varnish composition comprising, in a cosmetically acceptable medium comprising an organic solvent medium:
- at least one block copolymer of formula (I) below:
HO-(CH2-CH2-O)n-(CxH2xO)m-(CH2-CH2-O)p-H
in which n, m and p are, independently, integers ranging from 1 to 1000 and x is equal to 3; the said block copolymer being present in a content of at least 0.1% by weight relative to the total weight of the composition, the said block copolymer being a polyethylene glycol/polypropylene glycol/polyethylene glycol triblock polycondensate, and
- at least one thickener.

2. Composition according to Claim 1, **characterized in that** the block copolymer is present in an amount ranging from 0.1% to 15% by weight, preferably from 0.5% to 10% and better still from 1% to 8% by weight relative to the total weight of the composition.

3. Composition according to either of the preceding claims, **characterized in that** the organic solvent medium comprises at least one organic solvent chosen from:
- ketones that are liquid at room temperature, such as methyl ethyl ketone, methyl isobutyl ketone, diisobutyl ketone, isophorone, cyclohexanone or acetone;
- alcohols that are liquid at room temperature, such as ethanol, isopropanol; diacetone alcohol, 2-butoxyethanol or cyclohexanol;
- propylene glycol ethers that are liquid at room temperature, such as propylene glycol monomethyl ether, propylene glycol monomethyl ether acetate or dipropylene glycol mono-n-butyl ether;
- cyclic ethers such as γ-butyrolactone;
- short-chain esters (containing from 3 to 8 carbon atoms in total) such as ethyl acetate, methyl acetate, propyl acetate, isopropyl acetate, n-butyl acetate, isopentyl acetate, methoxypropyl acetate or butyl lactate;
- ethers that are liquid at room temperature, such as diethyl ether, dimethyl ether or dichlorodiethyl ether;
- alkanes that are liquid at room temperature, such as decane, heptane, dodecane or cyclohexane;
- alkyl sulfoxides such as dimethyl sulfoxide; aldehydes that are liquid at room temperature, such as benzaldehyde or acetaldehyde;
- ethyl 3-ethoxypropionate;
- carbonates such as propylene carbonate or dimethyl carbonate;
- acetals such as methylal;
- and mixtures thereof.

4. Composition according to one of the preceding claims, **characterized in that** the organic solvent medium represents from 10% to 95% by weight, preferably from 15% to 80% by weight and better still from 20% to 60% by weight relative to the total weight of the composition.

5. Composition according to one of the preceding claims, **characterized in that** the thickener is chosen from silicas, especially hydrophobic silicas, clays such as montmorillonite, modified clays such as bentones, and polysaccharide alkyl ethers, and mixtures thereof.

6. Composition according to one of the preceding claims, **characterized in that** the thickener is present in a content ranging from 0.01% to 15% by weight, preferably from 0.1% to 10% and better still from 0.1% to 15% by weight relative to the total weight of the composition.

7. Composition according to one of the preceding claims **characterized in that** the total content of thickeners is less than or equal to 5% by weight and preferably less than or equal to 3% by weight relative to the total weight of the composition.

8. Composition according to one of the preceding claims, **characterized in that** it comprises a thickener chosen from bentones, in a content of less than or equal to 2% by weight relative to the total weight of the composition.

9. Composition according to one of the preceding claims, **characterized in that** it comprises a film-forming polymer.

10. Composition according to the preceding claim, **characterized in that** the film-forming polymer is present in a content ranging from 0.1% to 60% by weight, preferably ranging from 2% to 40% by weight and better still from 5% to 25% by weight relative to the total weight of the composition.

11. Composition according to one of the preceding claims, **characterized in that** it comprises a dyestuff.

12. Composition according to the preceding claim, **characterized in that** the dyestuff is present in a content ranging from 0.01% to 50% by weight and preferably from 0.01% to 30% by weight relative to the weight of the composition.

13. Non-therapeutic cosmetic process for making up or caring for the nails, comprising the application to the nails of at least one coat of a nail varnish composition according to one of Claims 1 to 12.

14. Cosmetic assembly comprising: i) a container delimiting at least one compartment, the said container being closed by means of a closing member, and ii) a composition placed inside the said compartment, the composition being in accordance with any one of Claims 1 to 12.

15. Cosmetic assembly according to Claim 14, **characterized in that** the container is at least partly made of grass.

16. Cosmetic assembly according to Claim 14, **characterized in that** the container is at least partly made of at least one material other than glass, for example a thermoplastic or a metal.

17. Assembly according to any one of Claims 14 to 16, **characterized in that,** in the closed position of the container, the closing member is screwed onto the container.

18. Assembly according to any one of Claims 14 to 16, **characterized in that**, in the closed position of the container, the closing member is coupled to the container other than by screwing, especially by click-fastening.

19. Assembly according to any one of Claims 14 to 18, **characterized in that** it comprises an applicator in the form of a brush comprising at least one tuft of bristles.

20. Assembly according to any one of Claims 14 to 18, **characterized in that** it comprises an applicator other than a brush, such as a spatula or a foam tip.

## Patentansprüche

1. Nagellackzusammensetzung, die in einem kosmetisch akzeptablen, ein organisches Lösemittelmedium enthaltenden Medium enthält:
mindestens ein Blockcopolymer der folgenden Formel (I):
HO-(CH₂-CH₂-O)ₙ-(CₓH₂ₓO)ₘ-(CH₂-CH₂-O)ₚ-H
wobei n, m und p unabhängig voneinander ganze Zahlen im Bereich von 1 bis 1000 bedeuten und x 3 ist,
wobei das Blockcopolymer in einer Menge von mindestens 0,1 Gew.-%, bezogen auf das Gesamtgewicht der Zusammensetzung, enthalten ist,
wobei das Blockcopolymer ein Dreiblockpolykondensat Polyethylenglycol/Polypropylenglycol/Polyethylenglycol ist,
und mindestens ein Verdickungsmittel.

2. Zusammensetzung nach Anspruch 1, **dadurch gekennzeichnet, dass** das Blockcopolymer in einer Menge von 0,1 bis 15 Gew.-%, bezogen auf das Gesamtgewicht der Zusammensetzung, vorzugsweise 0,5 bis 10 % und besser 1 bis 8 % enthalten ist.

3. Zusammensetzung nach einem der vorhergehenden Ansprüche,
**dadurch gekennzeichnet, dass** das organische Lösemittelmedium ein organisches Lösemittel enthält, das ausgewählt ist unter:
bei Raumtemperatur flüssigen Ketonen, wie Methylethylketon, Methylisobutylketon, Diisobutylketon, Isophoron, Cyclohexanon, Aceton;
bei Raumtemperatur flüssigen Alkoholen, wie Ethanol, Isopropanol, Diacetonalkohol, 2-Butoxyethanol, Cyclohexanol; bei Raumtemperatur flüssigen Propylenglycolethern, wie Propylenglycolmonomethylether, Propylenglycolmonomethyletheracetat, Dipropylenglycolmono-*n*-butylether; cyclischen Ethern, wie γ-Butyrolacton;
kurzkettigen Estern (mit insgesamt 3 bis 8 Kohlenstoffatomen), wie Ethylacetat, Methylacetat, Propylacetat, Isopropylacetat, *n*-Butylacetat, Isopentylacetat, Methoxypropylacetat, Butyllactat;
bei Raumtemperatur flüssigen Ethern, wie Diethylether, Dimethylether oder Dichlordiethylether;
bei Raumtemperatur flüssigen Alkanen, wie Decan, Heptan, Dodecan, Cyclohexan;
Alkylsulfoxiden, wie Dimethylsulfoxid;
bei Raumtemperatur flüssigen Aldehyden, wie Benzaldehyd, Acetaldehyd;
Ethyl-3-ethoxypropionat;
Carbonaten, wie Propylencarbonat, Dimethylcarbonat; Acetalen, wie Methylal; deren Gemischen.

4. Zusammensetzung nach einem der vorhergehenden Ansprüche, **dadurch gekennzeichnet, dass** das organische Lösemittelmedium 10 bis 95 Gew.-%, bezogen auf das Gesamtgewicht der Zusammensetzung, vorzugsweise 15 bis 80 Gew.-% und besser 20 bis 60 Gew.-% ausmacht.

5. Zusammensetzung nach einem der vorhergehenden Ansprüche,
**dadurch gekennzeichnet, dass** das Verdickungsmittel unter den Kieselsäuren, insbesondere hydrophoben Kieselsäuren, Tonen, wie Montmorillonit, modifizierten Tonen, wie Bentonen, Alkyl-ethern von Polysacchariden und deren Gemischen ausgewählt ist.

6. Zusammensetzung nach einem der vorhergehenden Ansprüche, **dadurch gekennzeichnet, dass** das Verdickungsmittel in einer Menge von 0,01 bis 15 Gew,-%, bezogen auf das Gesamtgewicht der Zusammensetzung, vorzugsweise 0,1 bis 10% und besser 0,1 bis 15 % enthalten ist.

7. Zusammensetzung nach einem der Absprüche 1 bis 5, **dadurch gekennzeichnet, dass** die Gesamtmenge der Verdickungsmittel kleiner oder gleich 5 Ges.-%, bezogen auf das Gesamtgewicht der Zusammensetzung, und vorzugsweise kleiner oder gleich 3 Gew.-% ist.

8. Zusammensetzung nach einem der vorhergehenden Ansprüche, **dadurch gekennzeichnet, dass** sie ein Verdickungsmittel, das unter den Bentonen ausgewählt ist, in einer Menge kleiner oder gleich 2 Gew.-%, bezogen auf das Gesamtgewicht der Zusammensetzung, enthält.

9. Zusammensetzung nach einem der vorhergehenden Ansprüche,
**dadurch gekennzeichnet, dass** sie ein filmbildendes Polymer enthält.

10. Zusammensetzung nach dem vorhergehenden Anspruch, **dadurch gekennzeichnet, dass** das filmbildende Polymer in einer Menge von 0,1 bis 60 Gew.-%; bezogen auf das Gesamtgewicht der Zusammensetzung, vorzugsweise 2 bis 40 Gew.-% und besser 5 bis 25 Gew.-% enthalten ist.

11. Zusammensetzung nach einem der vorhergehenden Ansprüche,
**dadurch gekennzeichnet, dass** sie ein Farbmittel enthält.

12. Zusammensetzung nach dem vorhergehenden Anspruch, **dadurch gekennzeichnet, dass** das Farbmittel in einer Menge von 0,01 bis 50 Gew.-%, bezogen auf das Gesamtgewicht der Zusammensetzung, und vorzugsweise 0,01 bis 30 % enthalten ist.

13. Kosmetisches Verfahren zum Schminken oder für die nichttherapeutische Pflege der Nägel, das das Auftragen mindestens eine Schicht einer Nagellackzusammensetzung nach einem der Ansprüche 1 bis 12 auf die Nägel umfasst.

14. Kosmetische Einheit umfassend: i) einen Behälter, der mindestens eine Abteilung abgrenzt, wobei der Behälter durch eine Verschlusseinrichtung verschlossen ist, und ii) eine Zusammensetzung, die im Innern der Abteilung angeordnet ist, wobei die Zusammensetzung einem der Ansprüche 1 bis 12 entspricht.

15. Kosmetische Einheit nach Anspruch 14, **dadurch gekennzeichnet, dass** der Behälter zumindest zum Teil aus Glas gebildet ist.

16. Kosmetische Einheit nach Anspruch 14, **dadurch gekennzeichnet, dass** der Behälter zumindest zum Teil aus einem von Glas verschiedenen Material gebildet ist, beispielsweise einem thermoplastischen Material oder einem Metall.

17. Einheit nach einem der Ansprüche 14 bis 16, **dadurch gekennzeichnet, dass** in der geschlossenen Stellung des Behälters die Verschlusseinrichtung mit dem Behälter verschraubt ist.

18. Einheit nach einem der Ansprüche 14 bis 16, **dadurch gekennzeichnet, dass** in der geschlossenen Stellung des Behälters die Verschlusseinrichtung an den Behälter auf eine andere Weise als durch Verschrauben gekoppelt ist, insbesondere durch Einrasten.

19. Einheit nach einem der Ansprüche 14 bis 18, **dadurch gekennzeichnet, dass** sie einen Applikator in Form eines Pinsels umfasst, der mindestens ein Haarbüschel aufweist.

20. Einheit nach einem der Ansprüche 14 bis 18, **dadurch gekennzeichnet, dass** sie einen von einem Pinsel verschiedenen Applikator umfasst, wie einen Spatel oder einen Schaumapplikator.
